(19) Europäisches Patentamt
European Patent Office
Office européen des brevets

(11) EP 1 673 403 B1

(12) **EUROPEAN PATENT SPECIFICATION**

(45) Date of publication and mention of the grant of the patent:
**23.12.2009 Bulletin 2009/52**

(21) Application number: **04794315.4**

(22) Date of filing: **06.10.2004**

(51) Int Cl.:
***C08G 61/12*** *(2006.01)*

(86) International application number:
**PCT/US2004/032914**

(87) International publication number:
**WO 2005/035618 (21.04.2005 Gazette 2005/16)**

(54) **TELECHELIC EMISSIVE OLIGOMERS AND POLYMERS DERIVED THEREFROM**

TELECHELE EMITTIERENDE OLIGOMERE UND DAVON ABGELEITETE POLYMERE

OLIGOMERES EMISSIFS, TELECHELIQUES ET POLYMERES DERIVES DE CES DERNIERS

(84) Designated Contracting States:
**AT BE BG CH CY CZ DE DK EE ES FI FR GB GR HU IE IT LI LU MC NL PL PT RO SE SI SK TR**

(30) Priority: **07.10.2003 US 680470**

(43) Date of publication of application:
**28.06.2006 Bulletin 2006/26**

(73) Proprietor: **GENERAL ELECTRIC COMPANY**
**Schenectady, NY 12345 (US)**

(72) Inventor: **CELLA, James, A.**
**Clifton Park, NY 12065 (US)**

(74) Representative: **Szary, Anne Catherine et al**
**London Patent Operation**
**GE International Inc.**
**15 John Adam Street**
**London**
**WC2N 6LU (GB)**

(56) References cited:
**US-A- 5 516 877 US-B1- 6 169 163**

- **SCHERF U., LIST E.J.W.: "Semiconducting Polyfluorenes - Towards Reliable Structure-Property Relationships" ADVANCED MATERIALS, vol. 14, no. 7, 2002, pages 477-487, XP002315008 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

BACKGROUND OF THE INVENTION

Field of the Invention

The present invention is directed to telechelic emissive, semi-conductive end-functionalized oligomers which can be polymerized by a variety of conventional techniques to afford emissive polymers. The polymers are useful as active layers in light emitting as well as photovoltaic devices.

Discussion of the Art

Polyphenylenes, polyfluorenes and other conjugated aromatic polymers are well known as active layers in electroluminescent devices. See U. Scherf and E.J. List, Adv. Mater., 14 (7), 477 (2002), and M.T. Bernius, M. Inbasekaran, J. O'Brien and W. Wu, Adv. Mater., 12 (23), 1737 (2000). These polymeric materials are generally prepared using aromatic coupling reactions, such as the Suzuki or Stille coupling or the nickel catalyzed coupling reactions of aryl halides. Although the methodology for preparing these polymers has been well established, in many cases the coupling-polymerization reactions afford by-products that limit the molecular weight and may either quench fluorescence or induce significant red shifts in the emission spectrum, thus limiting the color tunability of the devices.

Electroluminescent polymers bearing linking groups have been prepared and converted to electroactive copolymers by various methods. See C. Schmitt, H.G. Nothofer, A. Falcou and U. Scherf, Macromol. Rapid Commun., 22, 624 (2001); T. Miteva, A. Meisel, W. Knoll, H.G. Nothofer, U. Scherf, D.C. Muller, K. Meerholz, A. Yasuda and D. Neher, Adv. Mater., 13 (8), 565 (2001); and R. Friend, J. Burroughs and D. Bradley, U.S. Patent No. 5,427,190 (1993) issued to Cambridge Display Technologies. The polymers in this invention are derived from phenol, thiophenol or aryl amine end-functional oligomers that can be converted into a variety of copolymers via these functional moieties. The oligomers or copolymers prepared according to the methods described are not believed to be known in the existing art.

BRIEF SUMMARY OF THE INVENTION

In accordance with a first aspect of the invention, a process to prepare end-functionalized conjugated oligomers of polyarylenes that can be polymerized via the reactive end-group is provided.

A further aspect of the invention relates to the reaction of a phenol, thiol or amine functional moiety, which is substituted with an aryl halide, with either a bis-boronic acid or ester or a bis-haloarene using Suzuki coupling conditions to form the end-functionalized conjugated oligiomers of polyarylenes.

Another aspect of the invention relates to polymerization of the end-functionalized conjugated oligiomers of polyarylenes prepared according to the invention by reacting the phenol, thiol or amine functional moiety of the oligiomer with a difunctional monomer to form the polymer.

An additional aspect of the invention relates to the telechlic emissive, semi-conductive end-functionalized oligiomers and polymers produced therefrom.

A further aspect of the invention relates to the use of the emissive polymers of the invention in the formation of films for use in, for example, light emitting and photovoltaic devices.

These and other aspects and objects of the invention will become apparent upon reading and understanding of the detailed description of the invention.

BRIEF DESCRIPTION OF THE DRAWINGS

The invention may take form in various components and arrangements of components, and in various steps and arrangement of steps.

The Figure illustrates, in a schematic format, one embodiment of a process for preparing the oligomers and corresponding polymers of the invention.

DETAILED DESCRIPTION OF THE INVENTION

The present invention is directed to an efficient process to prepare end-functionalized conjugated oligomers. There are a number of advantages to the approach described in this disclosure. For example, the telechelic oligomers can be readily purified by conventional techniques (chromatography, recrystallization, etc.). Also, the emissive components of the oligomers can be readily varied to achieve desirable processability characteristics, color emission, emission efficiency and charge transport properties. Further, the conjugation length is readily tailored and the polymerization

chemistry can be selected to minimize side reactions, maximize molecular weight control and tailor physical properties of the final polymer.

The oligomers are prepared by reaction of a phenol, thiophenol or amine functional moiety, appropriately substituted with an aryl halide, with either a bis-boronic acid or ester or a bis-haloarene respectively using Suzuki coupling conditions as described in N. Miyaura, T. Yanagi and A. Suzuki, Synth. Commun., (11) 513, (1981). The resulting oligomers can then be purified using conventional techniques. The purified oligomers may then be polymerized by reactions of the phenol, thiol or amine functional group with appropriate co-monomers to afford, for example, polycarbonates, polyesters, polyethers, polysilylethers, polyetherimides, polyimides. The resulting polymers can be isolated and fabricated into films or other media suitable for construction of devices such as light emitting devices and photovoltaic devices.

Additionally, the present invention is directed to polymers, and active layers of certain devices, such as light emitting devices, or photovoltaic devices, that are prepared from the process of the invention.

A schematic example of this concept is illustrated in the Figure. In this scheme, Ar represents any aromatic moiety including, but not limited to phenyl, substituted phenyl, naphthyl, anthryl, biphenylyl, etc., and any substituted variants of these species. "A" can be oxygen, sulfur or nitrogen, "n" is 1 or 2 depending on A. R may be, independently, alkyl, branched alkyl, hydrocarbon chains having 1 to about 40 carbon atoms, alkyl chains substituted with fluorine, cyano or aryl groups, aryl or substituted aryl. Additionally, Ar and the aryl ring bearing the AHn may be connected to each other by a carbon or carbon-heteroatom bond, such as in structures 1 and 2.

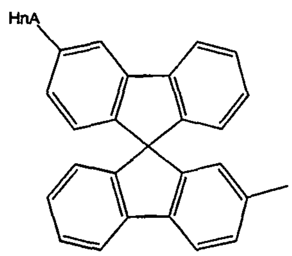

or

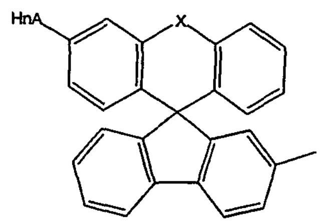

X=O, S, NR

1

2

Likewise, the R groups may be linked together as in structure 3.

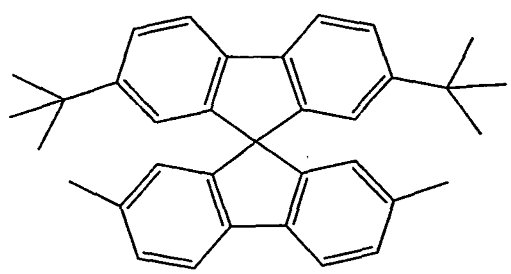

3

The AHn functional group used for linking these oligomers together may also be attached to other portions in this oligomer, such as in structure 4.

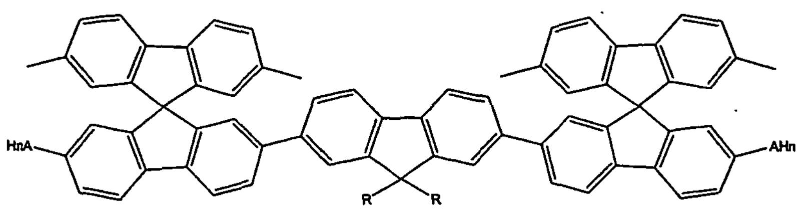

4

Additionally, the oligomer length can be extended as in the generic structure 5.

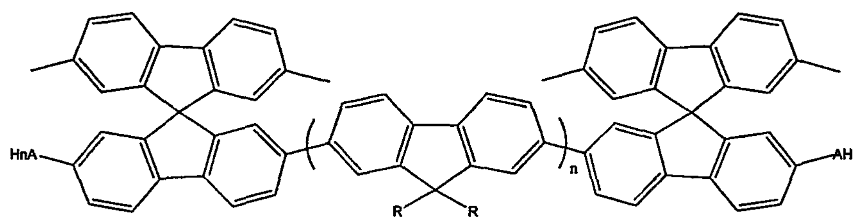

5

Further, some of the polymers described herein may also be obtained by first linking the AHn functional components together via a carbonate, or other suitable linkage, and then polymerizing through the arylhalide function using typical aryl coupling chemistry, such as described by Suzuki or Yamamoto previously referenced herein. Such a method would essentially be a reversal of the order of events to obtain the desired materials. This process is shown in the following scheme:

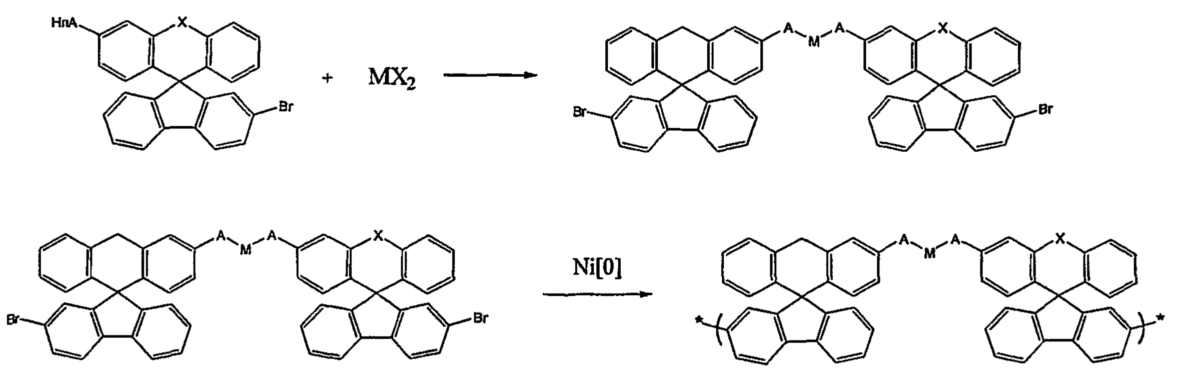


$MX_2$ is any difunctional monomer capable of reacting with AHn to form a homopolymer or copolymer. Examples of $MX_2$ include but are not limited to BPA-bis-chloroformate, terephthalic acid or its diacid chloride, dichlorophenylsulfone, pyromellitic dianhydride, adipoylchloride, diphenyldichlorosilane, dimethyldichlorosilane, 1,1,3,3-tetramethyldisiloxane, phosgene and the like, and mixtures thereof.

The "homopolymer" in this context means that the emissive segments are linked together solely by the linker $MX_2$. See, for example, structure 6. The "copolymer" in this context refers to structures wherein one or more emissive segments and/or a non-emissive segments are linked together by the link $MX_2$. The link segments in a copolymer can be dispersed randomly or in an alternating manner. The latter is referred to as an alternating copolymer, which is often abbreviated "alt-w-polymer." Structures 6 and 7 are illustrative of these types of structures:

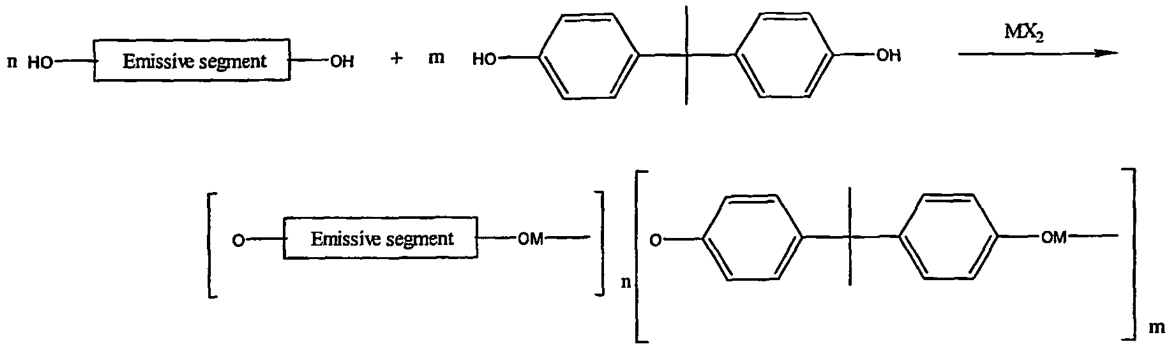


6 "random" copolymer

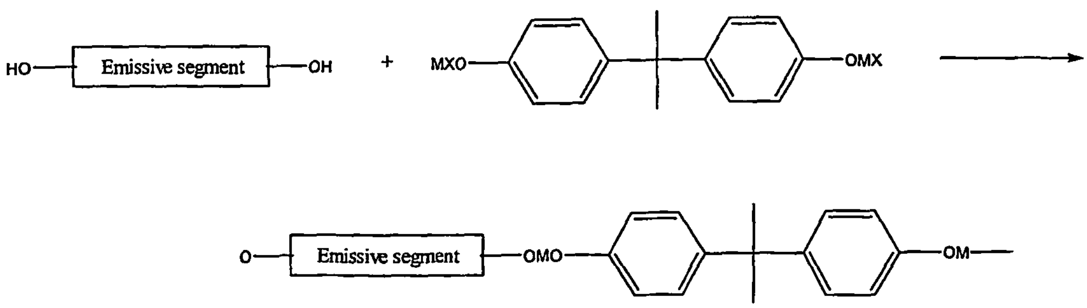

7 "alt-copolymer

In the schematic shown in the Figure, the starting material, a halogenated fluorenone such as 2-bromo-fluorenone (Ia), can be prepared by known procedures such as that described in J. Chem. Soc., p 1737, (1970) and by direct bromination of fluorenone in the presence of Fe and methylene chloride. Halogenation may take place at more than one position on the fluorenone ring system as in, for example, 2,7-dibromofluorenone.

The halogenated fluorenone (Ia) is then converted into intermediate reaction product (Ib) via a Grignard reaction. In the schematic shown in the Figure, a Grignard reactant ArMgX where Ar represents any aromatic moiety including, but not limited to, phenyl, substituted phenyl, naphthyl, anthryl, biphenylyl, etc. and any substituted variants thereof, and X is halogen, is reacted with the halogenated fluorenone (Ia) to produce an intermediate reaction product, such as the carbinol of formula (Ib) as shown in the Figure.

The carbinol of formula (Ib) is then reacted with a phenol, thiol or amine functional moiety. In the scheme of the Figure, the carbinol is reached with a phenyl ring having OH, SH, or $NH_3$ attached thereto which forms the compound of formula (Ic). While the compound (Ic) in the Figure has an aryl halide substitution, this derivative may be converted to an aryl boronic acid or ester prior to the coupling step.

The compound of formula (Ic) is then reacted with either a bis-boronic acid or ester or a bis-haloarene under conventional Suzuki coupling conditions as mentioned hereinbefore. In the reaction scheme of the Figure, a bis-boronic acid of Formula (Id) is reacted with the compound of Formula (Ic) in the presence of $(Ph_3P)_4$, $K_2CO_3$ and toluene to form the oligiomer of Formula (Ie)

The oligiomer of Formula (Ie) can then be polymerized by reacting the phenol, thiol or amine functional moiety with an appropriate co-monomer(difunctional monomer). The comonomer as shown in the reaction scheme of the Figure has a formula $MX_2$ and is for example, phosgene. As shown in the Figure, the resultant polymer is a compound of Formula (If).

In one embodiment a polymer of the general formula:

$$[(D\text{-}G_n\text{-}D)\text{-}M]_m$$

is formed in accordance with the present invention. D is preferably an "A-functional" segment of the general formula:

Ar-A-Ha

wherein Ar is an aromatic unit selected from the group consisting of phenyl, substituted phenyl, naphthyl, anthryl, biphenyl, substituted variants thereof, and mixtures thereof; A is selected from the group consisting of oxygen, nitrogen, sulfur, nil, and mixtures thereof; wherein "nil" represents a conjugation length of (D-$G_n$-G) that is interrupted by the A-M linkage and is an integer between about 1 and 3; G is preferably an oligophenylene, such as oligofluorene, as previously described; n is preferably an integer between about 1 and about 25, M is a linking group formed by reacting the emissive segment with a $MX_2$ linker as previously described, and m is an integer between about 1 and about 1000.

The following examples are meant for illustrative purposes only and are not intended to limited the invention to the particular embodiments described therein:

EXAMPLES

Example 1: Preparation of coumpound 1(f) from the Figure where R = n-hexyl, Ar = 4-t-butylphenyl, A= O and $MX_2$ = phosgene

A Grignard reagent prepared from 4.47 g. (21 mmol) of 4-t-butylbromobenzene and 0.753 g. (31 g-atoms) of magnesium plus 10 mmol of 1,2-dibromoethane in 50 ml of ether was treated over one hour with a hot solution of 5.18 g. (20 mmol) of 2-bromofluorenone in 25 ml. of toluene. The mixture was refluxed for one hour then quenched by addition of 50 ml. of saturated aqueous ammonium chloride. After separation and washing of the organic layer, solvent was evaporated to yield 10 g. of an oil that was chromotographed on 50 g. of silica gel (20% ethyl acetate-hexane as eluant). The product was a carbinol which was then isolated as an off-white solid in 92% yield. Reaction of this carbinol, (15.5 mmol) with 1.5 equivalents of phenol in methylene chloride (10 ml.) in the presence of

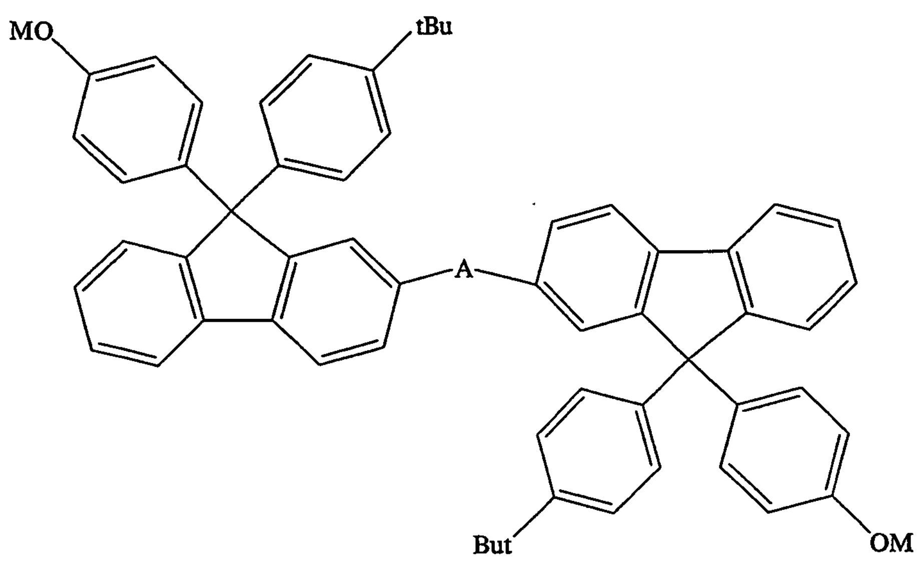

methanesulfonic acid (200μl) afforded 2-bromo-9-(4-t-butylphenyl)-9-(4-hydroxyphenyl)fluorine (61 %) yield. Reaction of the 2-bromo-9-(4-t-butylphenyl)-9-(4-hydroxyphenyl)fluorine compound with 0.5 equivalents of 9,9-dihexyl-2,7-bis-trimethyleneborate in a toluene/2M $K_2CO_3$ mixture in the presence of hexaethylguanidinium chloride (1mol%) and tetrakis-triphenylphosphine palladium(0) (1.6mol%) afforded the terfluorene bisphenol (68% yield). Mass spectrum: m/e 1110 (M*). Reaction of this bis-phenol with one equivalent of phosgene in methylene chloride in the presence of a pH

10 buffer afforded approximately an 80% yield of polymer 1(2) gpc (PS standards) Mw = 25025; Mn = 7808 Mw/Mn = 2.96.

Other functional monomers and polymers have been prepared using procedures similar to that described above. A summary of pertinent data for these materials is presented in Table 1.

Table 1. Pertinent data for oligomers and polymers

| A | M | Mw | Mn | Mw/Mn | Uv(max) | Emm(max) |
|---|---|---|---|---|---|---|
| 9,9-dihexylfluorene-2,7-diyl | H | 1,110 | 1,110 | 1.00 | 350 | 400, 425 |
| 9,9-dihexylfluorene-2,7-diyl | CO | 43,237 | 15,014 | 2.88 | 350 | 400,425 |
| 9,9-dihexylfluorene-2,7-diyl | CO/ (OBPAOCO) | 35,818 | 15,273 | 2.34 | 350 | 400,425 |
| 9,9-dihexylfluorene-2,7-diyl | COOBPAOCO | 20,098 | 8464 | 2.37 | 354 | |
| 9,9-dihexylfluorene-2,7-diyl | $Ph_2Si$ | <15,000 | <5,000 | - | - | |
| --- | H | 778 | 778 | 1.00 | 334 | |
| -- | COOBPAOCO | 42,917 | 9307 | 4.61 | 333 | |
| Oxa[a] | H | 998 | 998 | 1.00 | 347 | |
| Oxa[a] | COOBPAOCO | 28,547 | 12,309 | 2.32 | | |
| 9,10-anthryl | H | 954 | 954 | 1.00 | 382,407 | |
| 9,10-anthryl | COOBPAOCO | 31,942 | 13,420 | 2.38 | 383,399 | |
| 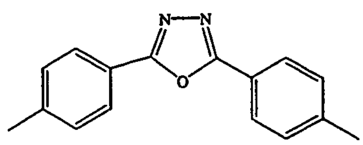 a= | | | | | | |

In addition to the above, the following tables 2 and 3 illustrate other monomeric and polymeric structures of compounds within the scope of the invention, a number of physical properties associated with the monomers and polymers are also represented in the tables below.

Table 2. Monomers

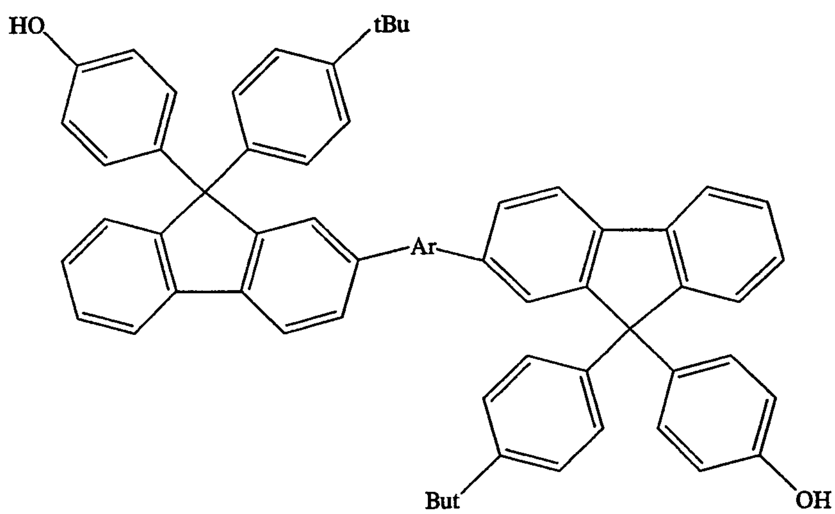

(continued)

| Ar | MW | mp °C | $8_{max}$(abs) (nm) | $8_{max}$(em) (nm) | Nem | ΔE(uv) (eV) |
|---|---|---|---|---|---|---|
| 2.1 | 1110 | 198-220 | 350 | 398,416 | 0.93 | 3.11 |
| 2.2 | 954 | 222-232 | 382 | 442 | 0.39 | 2.87 |
| 2.3 | 998 | 235-258 | 347 | 393,410 | 1.08 | 3.19 |
| | 778 | 230-235 | 334 | 416 | 0.93 | 3.08 |

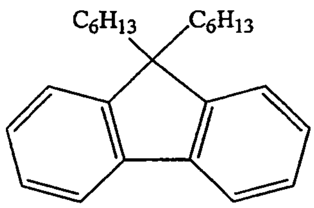

2.1

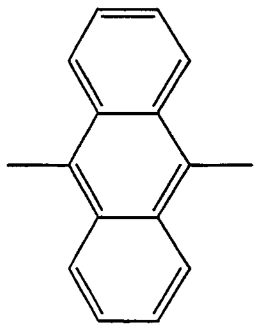

2.2

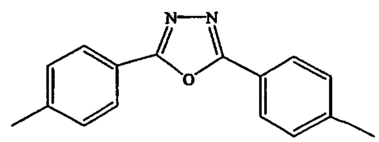

2.3

Table 3. Polymers

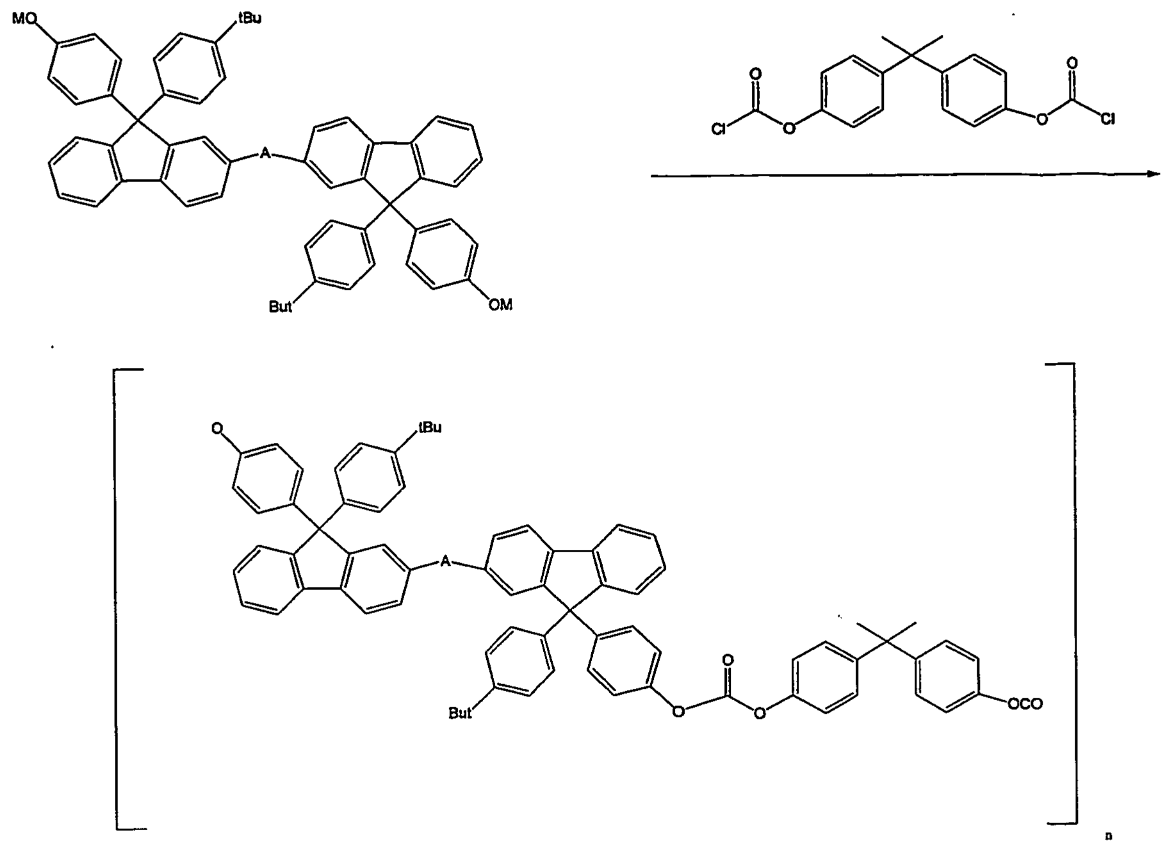

| Ar | MW | Mn | $8_{max}$(abs) (nm) | $8_{max}$(em) (nm) | Nem | ΔE(uv) (eV) |
|---|---|---|---|---|---|---|
| 3.1 | 20,098 | 8464 | 354 | 398,416 | 0.86 | 3.14 |
| 3.2 | 31,942 | 13,420 | 383,399 | 439 | 0.39 | 2.86 |
| 3.3 | 28,547 | 12,309 | 347 | 393,410 | 1.06 | 3.19 |

(continued)

| Ar | MW | Mn | $8_{max}$(abs) (nm) | $8_{max}$(em) (nm) | Nem | ΔE(uv) (eV) |
|---|---|---|---|---|---|---|
| | 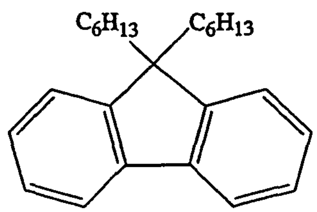 | | 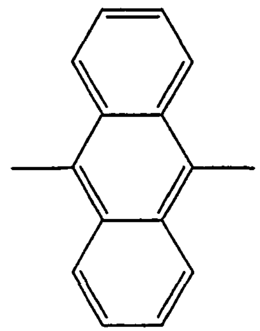 | | 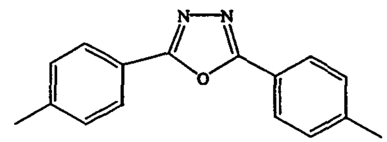 | |
| --- | 42,917 | 9307 | 333 | 387 | - | 3.35 |
| 3.1 | | | 3.2 | | 3.3 | |

Additionally, several polymers were prepared according to the following scheme:

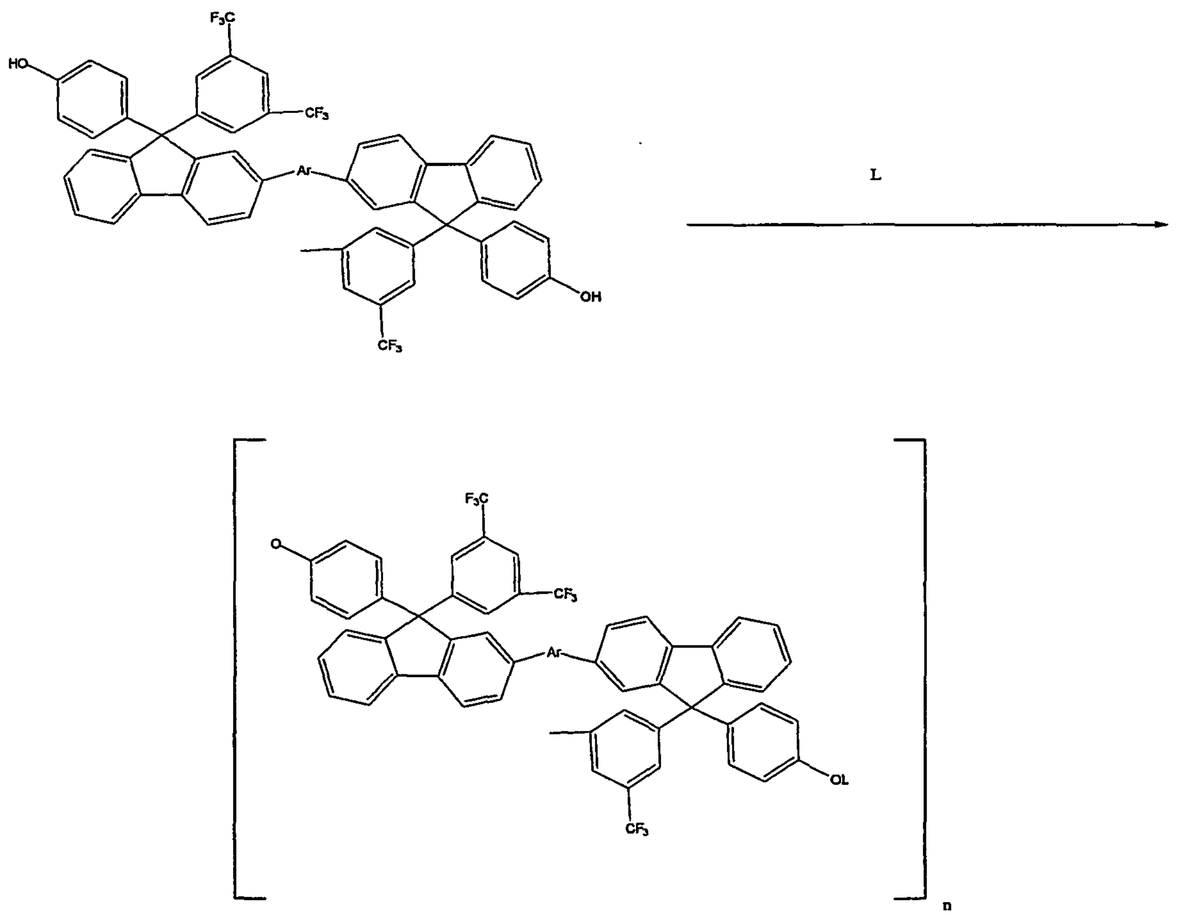


wherein Ar is:

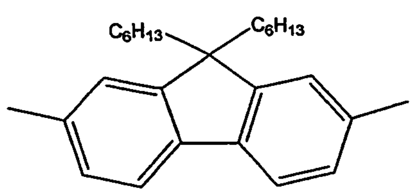

4.1

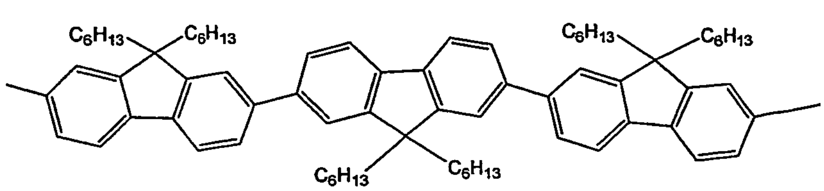

4.2

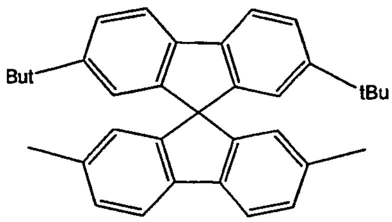

4.3

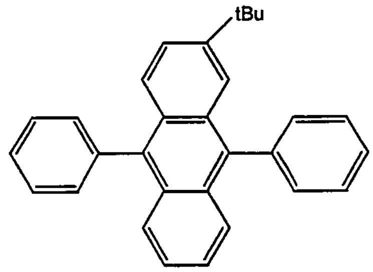

4.4

and L is:

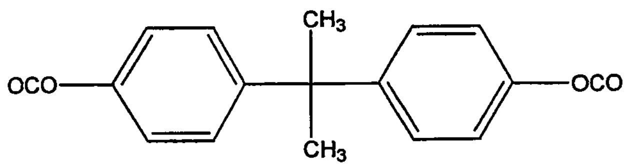

Polymers were also prepared according to the following scheme:

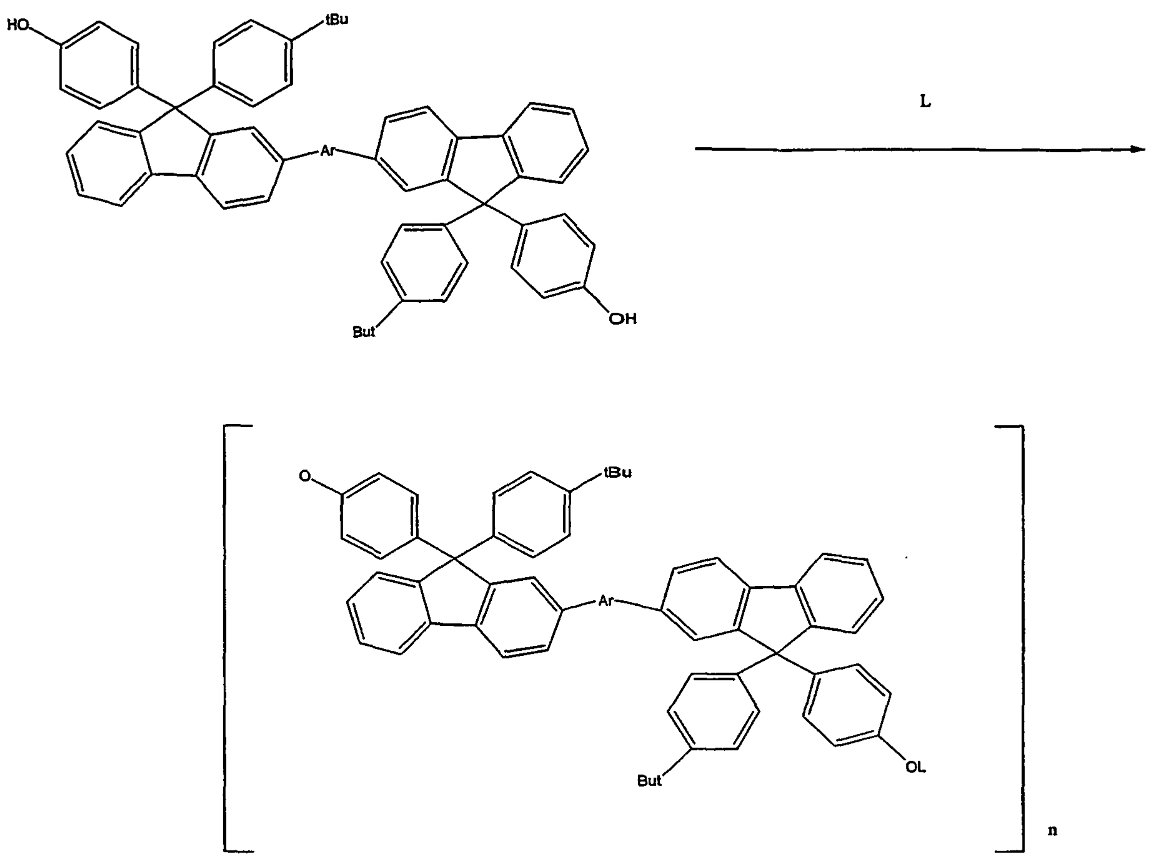


wherein Ar is the same as 2.1 previously set forth herein, and L is:

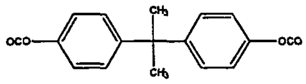

5.1

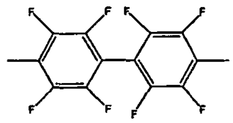

5.2

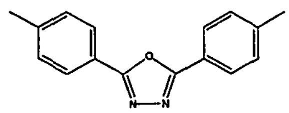

5.3

Additionally, with respect to the foregoing scheme, a polymer was formed using a co-monomer and structure 4.1. The co-monomer had the following structure:

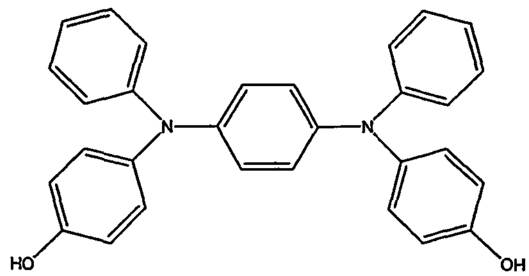

Several polymers were prepared and tested in a device having the layered structure ITO/PEDOT/X/LiF/Al were X is the polymer according to the invention. The X polymer was prepared by reacting on oligiomer of the formula:

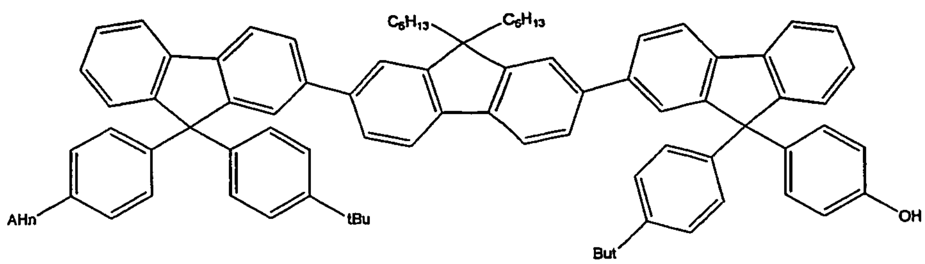

with a difunctional monomer as defined in the Table 4 below.

Table 4. Device Data

| Difunctional Monomer | Turn on V | W/W @20mA /cm$^2$ | LPWr | CIEx | CIEy |
|---|---|---|---|---|---|
| Homo-PC | 6.05 | 0.062% | 229 | 0.222 | 0.297 |
| Co-BPA PC | 6.9 | 0.019% | 170 | 0.208 | 0.228 |
| Alt-BPA PC | | - | - | - | - |
| Diphenylsilylether | 5.4 | .025% | 335 | 0.176 | 0.143 |
| CDTF1 | 3.11 | 0.23 | 130 | 0.173 | 0.138 |

In table 4, turn on V is the voltage at which the device emits visible light, W/W@20mA/cm$^2$, LPWr is lumens per watt, CIEx and CIEy are coordinates describing the color of the emitted light.

While the invention has been described herein relative to its preferred embodiments, it is of course contemplated that modifications of, and alternatives to, these embodiments, such modifications and alternatives obtaining the advantages and benefits of this invention, will be apparent to those of ordinary skill in the art having reference to this specification. It is contemplated that such modifications and alternatives are within the scope of this invention as subsequently claimed herein.

## Claims

1. A process for preparing an end-functionalized conjugated oligomer of a polyarylene comprising the steps of:

   a) effecting a reaction between a compound comprising a phenol, thiol or amine functional moiety which is substituted with an aryl halide, and having the formula:

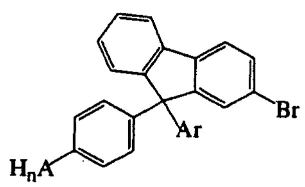

   wherein Ar is an aromatic moiety, A is O, S or N and n is 1 or 2; and a compound selected from a bis-boronic acid or ester or a bis-haloarene to form an oligomer;
   b) isolation and purification of the oligomer.

2. A process for forming a polymer comprising a series of end functionalized conjugated oligomers of a polyarylene comprising the steps of:

   a) effecting a reaction between a compound comprising a phenol, thiol or amine functional moiety which is substituted with an aryl halide, and having the formula:

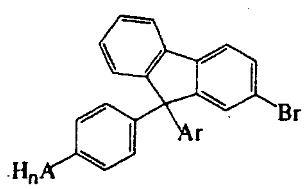

   wherein Ar is an aromatic moiety, A is O S or N and n is 1 or 2; and a compound selected from a bis-boronic acid or ester or a bis-haloarene to form an oligiomer having a phenol, thiol or amine functional moiety formed thereon;
   b) isolation and purification of the oligomer having a phenol, thiol or amine functional moiety formed thereon; and
   c) reacting the phenol, thiol or amine functional moiety present on the oligiomer of step b) with a difunctional monomer to form a polymer.

3. The process of claims 1 or 2 wherein the phenol, thiol or amine functional moiety is reacted with a compound having the formula:

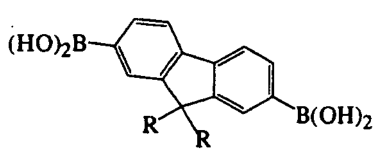

   wherein each R is selected from the same or different moiety selected from straight or branched alkyl, substitute alkyl, aryl or substituted aryl radicals.

4. The process of claim 3 wherein the resulting oligomer has the formula:

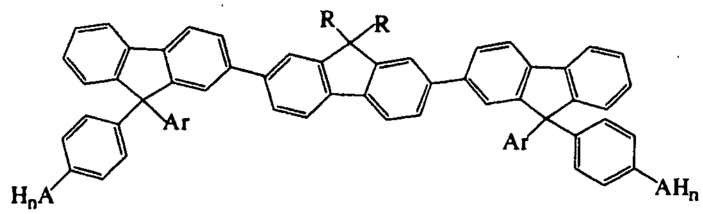

wherein Ar is an aromatic moiety, A is O, S or N, n = 1 or 2 and each R is selected from the same or different moiety selected from straight or branched alkyl, substituted, straight or branched alkyl, aryl or substituted aryl.

5. The process of claims 1, 2 or 4 wherein the Ar aromatic moiety is selected from phenyl, substituted phenyl, naphthyl, substituted naphthyl, anthryl, substituted anthryl biphenylyl, and substituted biphenylyl.

6. The process of claim 5 wherein Ar is selected from 4-t-butylphenyl.

7. The process of claim 4 wherein the oligomer is further polymerized with a difunctional monomer having the formula $MX_2$ to form a polymeric compound having the formula:

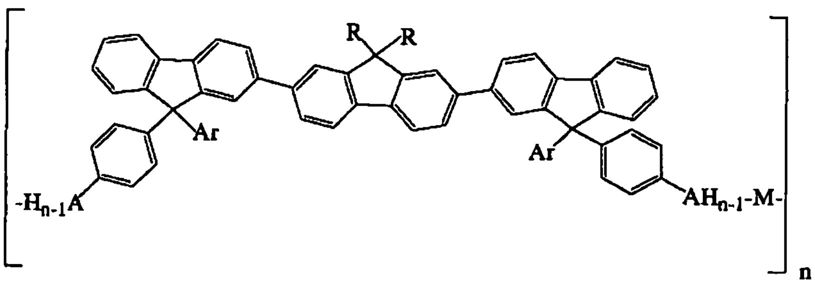

where $MX_2$ is a difunctional monomer which is capable of reacting with the AHn group to form a polymer.

8. The process of claim 2 or 7 wherein the difunctional monomer or the $MX_2$ difunctional monomer is a compound selected from BPA-bis-chloroformate, terephthalic acid, terephthalic diacid chloride, dichlorophenylsulfone, pyromellitic dianhydride, adipolychloride, diphenyldichlorosilane, dimethyldichlorosilane, phosgene, 1,1,3,3-tetramethyldisiloxane, and mixtures thereof.

## Patentansprüche

1. Verfahren zum Herstellen eines konjugierten Oligomers eines Polyarylens mit funktionellen Endgruppen, umfassend die Stufen:

a) Bewirken einer Umsetzung zwischen einer Verbindung, die eine funktionelle Phenol-, Thiol- oder Amingruppierung aufweist, die mit einem Arylhalogenid substituiert ist und die Formel aufweist:

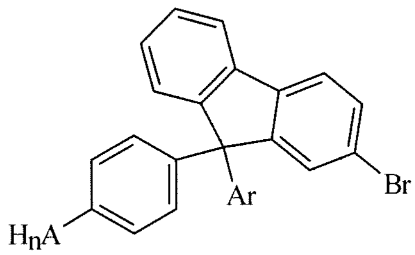

worin Ar eine aromatische Gruppierung ist, A O, S oder N ist und n 1 oder 2 ist und
einer Verbindung, ausgewählt aus einer Bis-boronsäure oder einem -ester oder einem Bis-halogenaren zur Bildung eines Oligomers,
b) Isolation und Reinigung des Oligomers.

**2.** Verfahren zum Bilden eines Polymers, umfassend eine Reihe konjugierter Oligomerer eines Polyarylens mit funktionellen Endgruppen, umfassend die Stufen:

a) Bewirken einer Umsetzung zwischen einer Verbindung, die eine funktionelle Phenol-, Thiol- oder Amingruppierung aufweist, die mit einem Arylhalogenid substituiert ist und die Formel aufweist:

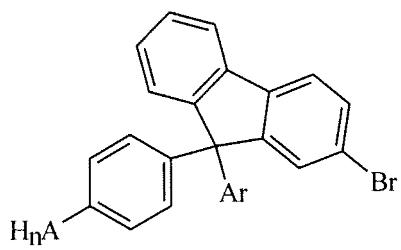

worin Ar eine aromatische Gruppierung ist, A O, S oder N ist und n 1 oder 2 ist und
einer Verbindung, ausgewählt aus einer Bis-boronsäure oder einem -ester oder einem Bis-halogenaren zur Bildung eines Oligomers, das eine darauf gebildete funktionelle Phenol-, Thiol- oder Amingruppierung aufweist,
b) Isolation und Reinigung des Oligomers, das eine darauf gebildete funktionelle Phenol-, Thiol- oder Amingruppierung aufweist und
c) Umsetzen der funktionellen Phenol-, Thiol- oder Amingruppierung, die auf dem Oligomer von Stufe b) vorhanden ist, mit einem difunktionellen Monomer, um ein Polymer zu bilden.

**3.** Verfahren nach Anspruch 1 oder 2, worin die funktionelle Phenol-, Thiol- oder Amingruppierung mit einer Verbindung der Formel umgesetzt wird:

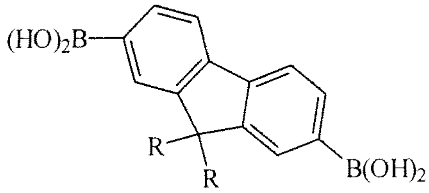

worin R ausgewählt ist aus der gleichen oder anderen Gruppierung, ausgewählt aus geradkettigen oder verzweigten Alkyl-, substituierten Alkyl-, Aryl- oder substituierten Arylresten.

4. Verfahren nach Anspruch 3, worin das resultierende Oligomer die Formel hat:

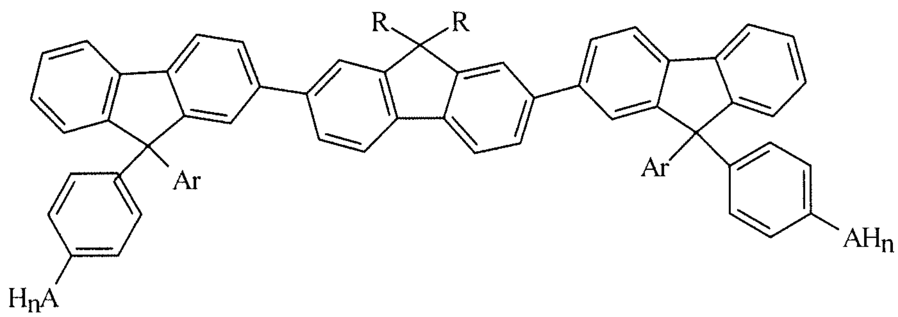

worin Ar eine aromatische Gruppierung ist, A O, S oder N ist und n 1 oder 2 ist und jedes R ausgewählt ist aus der gleichen oder anderen Gruppierung, ausgewählt aus geradkettigen oder verzweigten Alkyl-, substituierten geradkettigen und verzweigten Alkyl-, Aryl- oder substituierten Arylresten.

5. Verfahren nach Anspruch 1, 2 oder 4, worin die aromatische Gruppierung Ar ausgewählt ist aus Phenyl, substituiertem Phenyl,. Naphthyl, substituiertem Naphthyl, Anthryl, substituiertem Anthryl, Biphenylyl und substituiertem Biphenylyl,

6. Verfahren nach Anspruch 5, worin Ar ausgewählt ist aus 4-t-Butylphenyl.

7. Verfahren nach Anspruch 4, worin das Oligomer weiter mit einem difunktionellen Monomer der Formel $MX_2$ polymerisiert wird, um eine polymere Verbindung der Formel zu bilden:

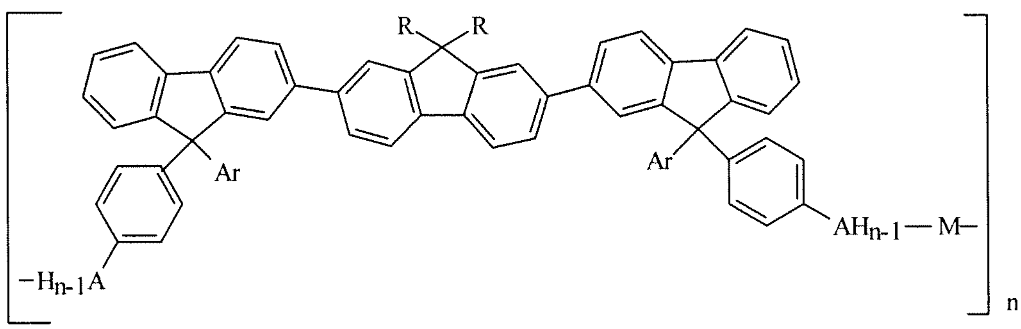

worin $MX_2$ ein difunktionelles Monomer ist, das in der Lage ist, mit der $AH_n$-Gruppe zur Bildung eines Polymers zu reagieren.

8. Verfahren nach Anspruch 2 oder 7, worin das difunktionelle Monomer oder das difunktionelle $MX_2$-Monomer eine Verbindung ist, ausgewählt aus BPA-bis-chlorformiat, Terephthalsäure, Terephthaldisäurechlorid, Dichlorphenylsulfon, Pyromellitsäuredianhydrid. Adipolychlorid, Diphenyldichlorsilan, Dimethyldichlorsilan, Phosgen, 1,1,3,3-Tetramethyldisiloxan und Mischungen davon.

**Revendications**

1. Procédé de préparation d'un oligomère conjugué à terminaison fonctionnalisée d'un polyarylène comprenant les étapes de :

a) mise en oeuvre d'une réaction entre un composé comprenant une fraction fonctionnelle phénol, thiol ou amine qui est substituée avec un halogénure d'aryle, et présentant la formule :

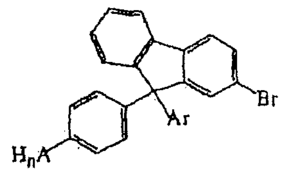

dans laquelle Ar est une fraction aromatique, A représente O, S ou N et n est égal à 1 ou 2 ; et un composé sélectionné à partir d'un acide ou ester bis-boronique ou d'un bis-haloarène afin de former un oligomère ;
b) isolement et purification de l'oligomère.

**2.** Procédé de formation d'un polymère comprenant une série d'oligomères conjugués à terminaison fonctionnalisée d'un polyarylène comprenant les étapes de :

a) mise en oeuvre d'une réaction entre un composé comprenant une fraction fonctionnelle phénol, thiol ou amine qui est substituée avec un halogénure d'aryle, et présentant la formule :

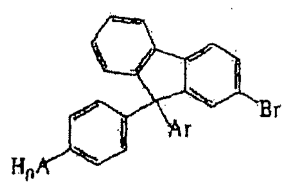

dans laquelle Ar est une fraction aromatique, A représente O, S ou N et n est égal à 1 ou 2 ; et un composé sélectionné à partir d'un acide ou ester bis-boronique ou d'un bis-haloarène afin de former un oligomère sur lequel est formé une fraction fonctionnelle phénol, thiol ou amine ;
b) isolement et purification de l'oligomère sur lequel est formé une fraction fonctionnelle phénol, thiol ou amine ; et
c) mise en réaction de la fraction fonctionnelle phénol, thiol ou amine présente sur l'oligomère de l'étape b) avec un monomère bifonctionnel afin de former un polymère.

**3.** Procédé selon les revendications 1 ou 2, dans lequel la fraction fonctionnelle phénol, thiol ou amine est mise en réaction avec un composé présentant la formule :

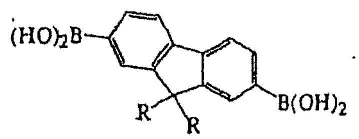

dans laquelle chaque R est sélectionné à partir de la même fraction ou d'une fraction différente sélectionnée à partir de radicaux alkyle ramifié ou linéaire, alkyle substitué, aryle ou aryle substitué.

**4.** Procédé selon la revendication 3, dans lequel l'oligomère résultant présente la formule :

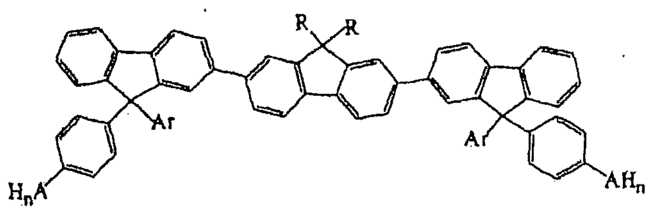

dans laquelle Ar est une fraction aromatique, A représente O, S ou N, n = 1 ou 2 et chaque R est sélectionné à partir de la même fraction ou d'une fraction différente sélectionnée à partir d'un alkyle ramifié ou linéaire, d'un alkyle substitué, d'un aryle ou d'un aryle substitué.

**5.** Procédé selon les revendications 1, 2 ou 4 dans lequel la fraction aromatique Ar est sélectionnée à partir du phényle, du phényle substitué, du naphtyle, du naphtyle substitué, de l'anthryl, du biphénylyle anthryl substitué, et du biphénylyle substitué.

**6.** Procédé selon la revendication 5 dans lequel Ar est sélectionné à partir du 4-t-butylphényle.

**7.** Procédé selon la revendication 4, dans lequel l'oligomère est de nouveau polymérisé avec un monomère bifonctionnel présentant la formule $MX_2$ afin de former un composé polymère présentant la formule :

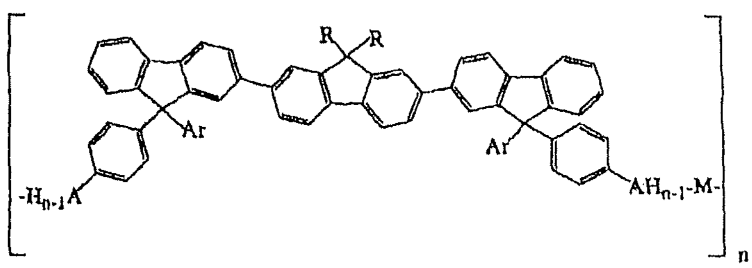

dans laquelle $MX_2$ représente un monomère bifonctionnel qui est capable de réagir avec le groupe AHn afin de former un polymère.

**8.** Procédé selon la revendication 2 ou 7, dans lequel le monomère bifonctionnel ou le monomère bifonctionnel $MX_2$ est un composé sélectionné à partir du BPA-bis-chloroformate, de l'acide téréphtalique, du chlorure de diacide téréphtalique, du dichlorophénylsulfone, du dianhydride pyromellitique, de l'adipolychlorure, du diphényldichlorosilane, du diméthyldichlorosilane, du phosgène, du 1,1,3,3-tétraméthyldisiloxane, et de leurs mélanges.

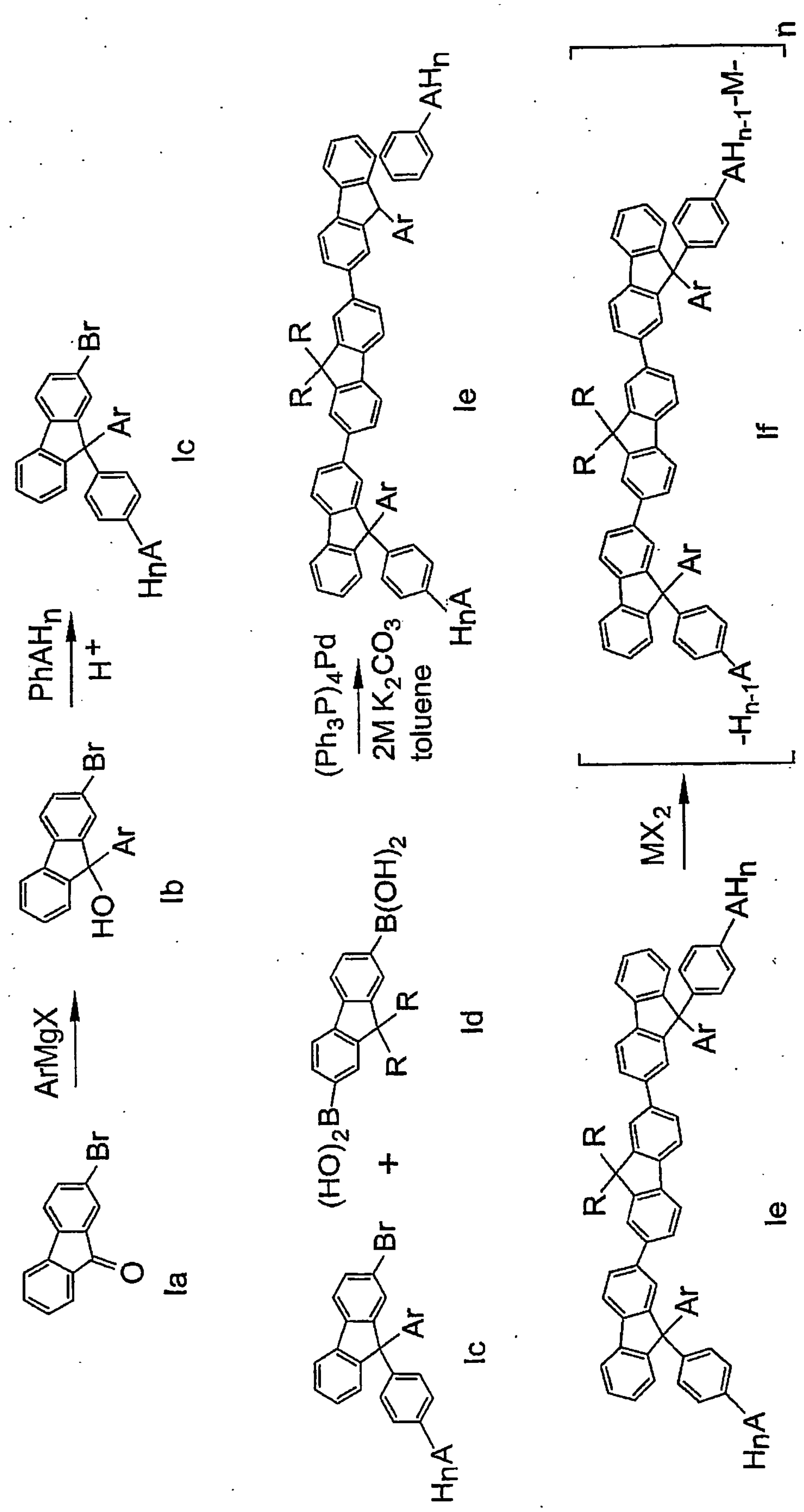
Ia
ArMgX
Ib
PhAH$_n$
H$^+$
Ic
Ic
+
Id
(Ph$_3$P)$_4$Pd
2M K$_2$CO$_3$
toluene
Ie
Ie
MX$_2$
If

Figure

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5427190 A, R. Friend, J. Burroughs and D. Bradley


### Non-patent literature cited in the description


- **U. Scherf ; E.J. List.** *Adv. Mater.,* 2002, vol. 14 (7), 477
- **M.T. Bernius ; M. Inbasekaran ; J. O'Brien ; W. Wu.** *Adv. Mater.,* 2000, vol. 12 (23), 1737
- **C. Schmitt ; H.G. Nothofer ; A. Falcou ; U. Scherf.** *Macromol. Rapid Commun.,* 2001, vol. 22, 624
- **T. Miteva ; A. Meisel ; W. Knoll ; H.G. Nothofer ; U. Scherf ; D.C. Muller ; K. Meerholz ; A. Yasuda ; D. Neher.** *Adv. Mater.,* 2001, vol. 13 (8), 565
- **N. Miyaura ; T. Yanagi ; A. Suzuki.** *Synth. Commun.,* 1981, 513
- *J. Chem. Soc.,* 1970, 1737